# EUROPÄISCHE PATENTSCHRIFT

(11) **EP 0 448 191 B1**
(45) Veröffentlichungstag und Bekanntmachung des Hinweises auf die Patenterteilung: **28.06.1995**
(21) Anmeldenummer: 91250081.6
(22) Anmeldetag: 18.03.1991
(51) Int. Cl.: C07D 257/02, C07F 5/00, A61K 49/00

(54) **1,4,7,10-Tetraazacyclododecan-butyltriole, Verfahren zu ihrer Herstellung und diese enthaltende pharmazeutische Mittel**
1,4,7,10-Tetraazacyclododecane-butyltriols, process for their preparation, and pharmaceutical agents containing these compounds
1,4,7,10-Tetraazacyclododécane-butyltriols, procédé pour leur préparation et agents pharmaceutiques les contenant

(30) Priorität: 19.03.1990 DE 4009119
(43) Veröffentlichungstag der Anmeldung: 25.09.1991
(73) Patentinhaber: SCHERING AKTIENGESELLSCHAFT, 13342 Berlin (DE)
(72) Erfinder: Platzek, Johannes, Dr., W-1000 Berlin 33 (DE); Gries, Heinz, Dr., W-1000 Berlin 31 (DE); Weinmann, Hanns-Joachim, Dr., W-1000 Berlin 38 (DE); Schuhmann-Giampieri, Gabriele, Dr., W-1000 Berlin 45 (DE); Press, Wolf-Rüdiger, W-1000 Berlin 42 (DE)

(56) Entgegenhaltungen:
- EP-A- 0 255 471
- EP-A- 0 292 689
- EP-A- 0 325 762

## Beschreibung

Die vorliegende Erfindung betrifft den in den Patentansprüchen gekennzeichneten Gegenstand, d.h. 1,4,7,10-Tetraazacyclododecan-butyltriole, deren Komplexe und Komplexsalze, diese Verbindungen enthaltende Mittel, ihre Verwendung als Diagnostika und Therapeutika sowie Verfahren zur Herstellung dieser Verbindungen und Mittel.

In der Europäischen Patentanmeldung 87730085.5 mit der Veröffentlichungsnummer 0 255 471 werden makrocyclische Verbindungen der allgemeinen Formel I 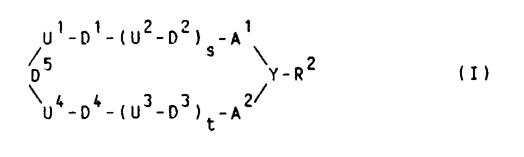
beansprucht,
worin
- Y: ein Stickstoff- oder Phosphoratom,
- A¹ und A²: gleich oder verschieden sind und jeweils eine geradkettige oder verzweigte Alkylengruppe mit 2 bis 6 Kohlenstoffatomen,
- U¹, U², U³, U⁴: gleich oder verschieden sind und jeweils eine direkte Bindung oder eine geradkettige oder verzweigte Alkylengruppe mit 1 bis 6 Kohlenstoffatomen,
- D¹, D², D³, D⁴: gleich oder verschieden sind und jeweils ein Sauerstoff- oder Schwefelatom, eine Alkylengruppe mit 1 bis 6 Kohlenstoffatomen oder eine Gruppe N-R⁷ mit R⁷ in der Bedeutung eines Wasserstoffatoms, einer geradkettigen oder verzweigten Alkylenkette mit 1 bis 4 Kohlenstoffatomen, die am Ende eine COOR¹-Gruppe trägt, wobei R¹ für ein Wasserstoffatom oder ein Metallionenäquivalent steht,
- D⁵: die für D¹, D², D³ und D⁴ angegebene Bedeutung hat sowie die Gruppe 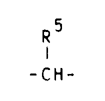 mit R⁵ in der Bedeutung eines Wasserstoffatoms oder einer gegebenenfalls Imino-, Phenylenoxy-, Phenylenimino-, Amid-, Estergruppe(n), Sauerstoff-, Schwefel- und/oder Stickstoff-Atom(e) enthaltenden gegebenenfalls durch Hydroxy-, Mercapto-, Imino- und/oder Aminogruppe(n) substituierten geradkettigen oder verzweigten, gesättigten oder ungesättigten C₁-C₂₀-Alkylengruppe, die am Ende entweder eine funktionelle Gruppe oder gebunden über diese ein Makromolekül B aufweist,
- s und t: ganze Zahlen von 0 bis 5,
- R²: Wasserstoff, eine gerade oder verzweigte, gesättigte oder ungesättigte, gegebenenfalls durch eine oder mehrere Hydroxy- oder niedere Alkoxygruppen substituierte Alkyl-, Acyl- oder Acylalkylgruppe mit 1 bis 16 Kohlenstoffatomen,
-CH₂-X-V mit X in der Bedeutung von Carbonyl, einer geradkettigen oder verzweigtkettigen Alkylengruppe mit 0 bis 10 Kohlenstoffatomen, die gegebenenfalls durch eine oder mehrere Hydroxy- oder niedere Alkoxygruppen substituiert ist oder einer gerad- oder verzweigtkettigen durch Sauerstoffatome unterbrochenen Alkylengruppe mit 2 bis 23 Kohlenstoffatomen,
- V: in der Bedeutung von 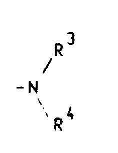 oder -COOR⁶, wobei R³ und R⁴ unabhängig voneinander Wasserstoff, eine gerade oder verzweigte, gegebenenfalls durch eine oder mehrere Hydroxy- oder niedere Alkoxygruppen substituierte Alkylgruppe mit 1 bis 16 Kohlenstoffatomen oder R³ und R⁴ gemeinsam mit dem Stickstoffatom einen gegebenenfalls ein weiteres Heteroatom enthaltenden gesättigten Fünf- oder Sechsring und R⁶ Wasserstoff oder einen gesättigten, ungesättigten, gerad- oder verzweigtkettigen oder cyclischen Kohlenwasserstoffrest mit bis zu 16 Kohlenstoffatomen oder eine Aryl- oder Aralkylgruppe darstellen, oder
- R² oder R³: einen über eine 2 bis 20 Kohlenstoffatome enthaltende Alkylenkette (K), die gegebenenfalls an den Enden Carbonylgruppen trägt und gegebenenfalls durch ein oder mehrere Sauerstoffatome oder R¹-Carboxymethyliminogrupen unterbrochen oder durch eine oder mehrere Hydroxy-, niedere Alkoxy- oder Carboxy-niederalkyl-Gruppen substituiert ist, gebundenen zweiten Makrozyklus der Formel I 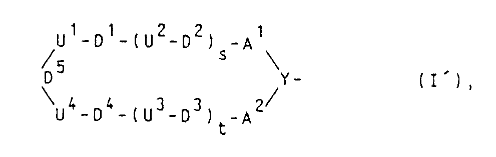 der anderer Struktur als der Grundkörper des ersten sein kann, oder
- R² B oder CH₂-COB: bedeuten,
mit der Maßgabe, daß, wenn R² für B oder CH₂-COB steht, R⁵ ein Wasserstoffatom bedeutet, daß mindestens zwei COOR¹-Gruppen im Molekül vorhanden sind und daß zwei Heteroatome des Makrocyclus jeweils über eine Alkylengruppe mit mindestens zwei Kohlenstoffatomen verbunden sind, und im Molekül vorhandene funktionelle Gruppen gewünschtenfalls mit Makromolekülen konjugiert und gewünschtenfalls freie Carboxylgruppen mit organischen oder anorganischen Basen oder Aminosäuren und basische Gruppen mit anorganischen oder organischen Säuren versalzt sind.

Die Substanzen und die aus ihnen bereiteten Lösungen erfüllen die an pharmazeutisch verwendbaren Chelate zu stellenden Anforderungen. Sie besitzen eine starke und durch die Wahl geeigneter Metallatome an die jeweiligen Prinzipien der diagnostischen oder therapeutischen Methode (Röntgen, NMR, Ultraschall, Nuklearmedizin) anpassungsfähige Wirksamkeit.

Unter der Vielzahl der in der EP-A 0 255 471 sowie auch in EP-A 0 292 689 beanspruchten Verbindungen weisen die 1,4,7,10-Tetraazacyclododecan-butylder allgemeinen Formel I_{A} 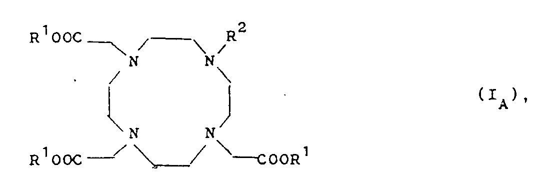
worin
- R¹: unabhängig voneinander Wasserstoff oder ein Metallionenäquivalent und
- R²: ein Butyltriol-Rest
bedeuten,
sowie deren Salze mit organischen oder anorganischen Basen oder Aminosäuren,
derart herausragende Eigenschaften auf, daß selbst im Vergleich zu der strukturell engst verwandten, in EP-A 0 255 471 offenbarten Verbindung (Beispiel 6) die Verwendung dieser ausgewählten Verbindungen einen wesentlichen Vorteil sicherstellt.

Verbindungen der allgemeinen Formel I_{A} mit R¹ in der Bedeutung von Wasserstoff werden als Komplexbildner und mit mindestens zwei der Substituenten R¹ in der Bedeutung eines Metallionenäquivalents als Metallkomplexe bezeichnet.

Das Element, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Für die Verwendung der erfindungsgemäßen Mittel in der Nuklearmedizin muß das Zentralion radioaktiv sein, Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Bevorzugte Reste R² sind der 2,3,4-Trihydroxybutyl- und der 1-Hydroxymethyl-2,3-dihydroxypropyl-Rest.

Wenn nicht alle aciden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins.

Als Beleg für die oben genannten überraschenden und hervorragenden Eigenschaften der erfindungsgemäßen Verbindungen sollen die Ergebnisse tierexperimenteller Untersuchungen zur Bestimmung der akuten intravenösen (LD₅₀) sowie der neuralen Verträglichkeit (ED₅₀) zweier erfindungsgemäßer Butyltriol-Verbindungen, d.h. von Verbindungen der allgemeinen Formel I_{A}, die in EP-A 0 255 471 weder beschrieben noch herausgestellt werden, im Vergleich zu derjenigen in EP-A 0 255 471 beschriebenen Verbindung, die den beiden Butyltriol-Makrocyclen strukturell nächstliegend ist, angeführt werden:

### 1. Akute Toxizitätsbestimmung (LD₅₀)

Im Einzelkäfig (Fa. Rhema/Hofheim) wurde Mäusen (Gewicht: 18 - 22 g) das Kontrastmittel annähernd körperwarm in eine Caudalvene mit einer Geschwindigkeit von 2 ml/min verabreicht.

Die Kontrastmittel wurden jeweils 3 Mäusen in 3 Dosierungen bei variablen Volumina und konstanter Konzentration verabreicht. Die Zuordnung der Tiere zu den einzelnen Dosierungen und die Injektionsreihenfolge der Dosen erfolgte zufällig.

Der Beobachtungszeitraum der Tiere erstreckte sich über 7 Tage p. injectionem. Als Kriterium der Wirksamkeit galt der Tod der Tiere.

### 2. Intracisternale Verabreichung (ED₅₀)

Einer gleichen Anzahl weiblicher und männlicher Ratten (130 - 170 g, Wistar-Han-Schering, SPF) wurde in leichter Äthernarkose die Prüfsubstanz einmalig intracisternal durch suboccipitale Punktion in konstanten volumina (0,08 ml/Tier) bei unterschiedlichen Konzentrationen verabreicht (3 Dosierungen a 10 Tiere pro Substanz). Eine Volumen-Stichkontrolle (Ringerlösung) wurde der Untersuchung angeschlossen. Als Wirkungskriterien wurden Haltungsanomalien, Krämpfe und der Tod der Tiere bewertet.

Die statistische Auswertung sämtlicher Untersuchungsergebnisse erfolgte mit der Probit-Analyse.

### 3. Ergebnisse

| Substanz (Gd-Komplex) | R² | akute i.v. LD₅₀ (mmol/kg) | neurale ED₅₀ (»mol/kg) |
|---|---|---|---|
| Beispiel 1 dieser Erfindung | -CH₂CH(OH)-CH(OH)CH₂OH | 35 | 27 |
| Beispiel 2 dieser Erfindung | -CH(CH₂OH)-CH(OH)-CH₂OH | 30 | 29 |
| Beispiel 6 EP-A 0 255 471 | -CH₂-CH(OH)-CH₂OH | 25 | 14 |

Kontrastmittel für die medizinische Diagnostik sollten hervorragend verträglich sein und sich biologisch möglichst inert verhalten. Monomere Kontrastmittel sollten möglichst der intravenösen Vertraglichkeit von z.B. Mannitol (d.h. 30 - 40 mmol/kg) nahekommen. Vergleichbare Verbindungen mit geringeren Werten sind daher biologisch nicht inert und zeigen eine unerwünschte Wechselwirkung mit dem Organismus. Das Propandiolderivat aus EP-A 0 255 471 zeigt mit einer akuten i.v. LD₅₀ von 25 mmol/kg deutlich niedrigere Werte als die beiden erfindungsgemäßen Verbindungen. Dieser relevante Unterschied zeigt eine gewisse Chemotoxizität der Vergleichssubstanz an. Die unerwünschte Wechselwirkung zeigte sich insbesondere beim Vergleich der Testsubstanzen in der neuralen Verträglichkeit. Die beiden erfindungsgemäßen Verbindungen zeigten eine deutlich bessere Verträglichkeit. Bei der Propandiol-Verbindung kam es bereits bei der Dosierung von 14 »mol/kg zu Veränderungen im Verhalten und deutlichen Krämpfen. Einige Tiere starben bei dieser Dosierung.

Bei den makrozyklischen Gadoliniumkomplexen dieser Erfindung konnte eine wesentlich bessere neurale Verträglichkeit beobachtet werden. Eine Beeinflussung des Verhaltens trat erst bei etwa doppelt so hohen Dosierungen wie bei der Vergleichssubstanz auf.

### Schlußfolgerung

Da die beiden erfindungsgemäßen Butyltriol-Verbindungen auch eine geringere Osmolalität (0,59 bzw. 0,57 gegenüber 0,62 osmol/kg; gemessen wurden wäßrige Lösungen der Gd-Komplexe in einer Konzentration von 0,5 mol/l) als die vorbeschriebene Propandiol-Verbindung zeigen, kann zusammenfassend festgestellt werden, daß die unerwünschten Wechselwirkungen dieser Verbindung mit dem biologischen Organismus bei den strukturell eng verwandten erfindungsgemäßen Verbindungen überraschenderweise nicht auftreten. Die beiden Butyltriol-Verbindungen zeigen deutliche Vorteile und sind daher als biologisch inerte Kontrastmittel wesentlich besser geeignet als die in EP-A 0 255 471 offenharten Verbindungen.

Die Herstellung der erfindungsgemäßen Verbindungen der allgemeinen Formel I_{A} erfolgt dadurch, daß man Verbindungen der allgemeinen Formel II 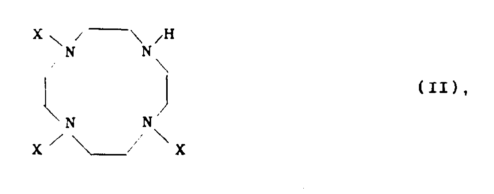
worin
- X: für eine Stickstoffschutzgruppe oder -CH₂COOY-Gruppe mit Y in der Bedeutung von Wasserstoff, eines Ammoniumkations, eines Alkalimetalls oder einer Schutzgruppe
steht,
mit einem den Rest R² in geschützter Form einführenden Substrat umsetzt,
die gegebenenfalls enthaltenen Stickstoffschutzgruppen X entfernt und die so freigesetzten -NH-Gruppen mit einem Essigsäure-Derivat der allgemeinen Formel III

HalCH₂COOY (III)

alkyliert,
worin Hal für Chlor, Brom oder Jod steht,
die (Hydroxy- und gegebenenfalls Säure-) Schutzgruppen entfernt und die so erhaltenen Verbindungen der allgemeinen Formel I_{A} mit R¹ in der Bedeutung von Wasserstoff mit einem Metalloxid oder Metallsalz in die Metallkomplexe der allgemeinen Formel I_{A} mit R¹ in der Bedeutung eines Metallionenäquivalents überführt und anschließend - falls gewünscht - noch vorhandene acide Wasserstoffatome durch Kationen anorganischer und/oder organischer Basen, Aminosäuren oder Aminosäureamide substituiert.

Als Säureschutzgruppen Y kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Säuren HalCH₂COOH können auch in Form ihrer Salze, vorzugsweise als Na- oder K-Salz, eingesetzt werden.

Die Abspaltung der Säureschutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Die drei Stickstoffatome des Edukts II tragen vor der den Rest R² einführenden Reaktion die Gruppe CH₂COOY oder Stickstoffschutzgruppen, zum Beispiel die Tosylat- oder Trifluoracetatgruppe, die vor der nachfolgenden Alkylierungsreaktion nach literaturbekannten Methoden abgespalten werden [die Tosylate z.B. mit Mineralsäuren, Alkalimetallen in flüssigem Ammoniak, Bromwasserstoffsäure und Phenol, RedAl^{(R)}, Lithiumaluminiumhydrid, NatriumAmalgam, vgl. z.B. Liebigs Ann. Chem. (1977), 1344, Tetrahedron Letters (1976), 3477; die Trifluoracetate z.B. mit Mineralsäuren oder Ammoniak in Methanol, vgl. z.B. Tetrahedron Letters (1967), 289].

Die N-Alkylierung mit einem Halogenessigsäure-Derivat der allgemeinen Formel III erfolgt in polaren aprotischen Lösungsmitteln wie zum Beispiel Dimethylformamid, Dimethylsulfoxid, Acetonitril, wäßriges Tetrahydrofuran oder Hexamethylphosphorsäuretriamid in Gegenwart eines Säurefängers wie zum Beispiel tertiäres Amin (zum Beispiel Triäthylamin, Trimethylamin, N,N-Dimethylaminopyridin, 1,5-Diazabicyclo[4.3.0]-nonen-5(DBN), 1,5-Diazabicyclo[5.4.0]-undecen-5-(DBU), Alkali- Erdalkalicarbonat, -hydrogencarbonat oder -hydroxid (zum Beispiel Lithium-, Natrium-, Magnesium-, Calcium-, Barium-, Kalium-, -carbonat, -hydroxid und -hydrogencarbonat) bei Temperaturen zwischen -10 °C und 120 °C, vorzugsweise zwischen 0 °C und 50 °C.

Als Hydroxyschutzgruppen kommen alle diejenigen infrage, die sich leicht einführen und später unter Rückbildung der letztlich gewünschten freien Hydroxygruppe auch wieder leicht abspalten lassen. Bevorzugte Schutzgruppen sind Ethergruppen wie zum Beispiel die Benzyl-, 4-Methoxybenzyl-, 4-Nitrobenzyl-, Trityl-, Di- und Triphenylmethyl-, Trimethylsilyl-, Dimethyl-t-butylsilyl-, Diphenyl-t-butylsilylgruppe. Bevorzugt sind die Hydroxygruppen jedoch in Form von Ketalen mit zum Beispiel Aceton, Acetaldehyd, Cyclohexanon oder Benzaldehyd geschützt.

Die Abspaltung der Hydroxyschutzgruppen erfolgt in an sich bekannter Weise, zum Beispiel im Falle eines Benzylethers durch reduktive Spaltung mit Lithium/Ammoniak oder durch hydrogenolytische Spaltung in Gegenwart von zum Beispiel Palladium-Kohle und im Falle einer Ether- oder Ketalspaltung durch Säurebehandlung mit Hilfe von zum Beispiel Kationenaustauschern, Trifluoressigsäure oder Mineralsäuren [siehe z.B. T.W. Greene "Protective Groups in Organic Synthesis", John Wiley and Sons (1981)].

Die Einführung des Restes R² erfolgt durch Alkylierung eines aus einer nucleofugen Gruppe wie zum Beispiel Cl, Br, J, CH₃C₆H₄SO₃, CF₃SO₃, CH₃SO₃ und des geschützten Restes R² aufgebauten Substrats oder eines Substrats, aus dem während der Reaktion intramolekular der gewünschte Rest R² generiert wird. Als Beispiel für den letztgenannten Fall seien die als z.B. Acetonide geschützten Hydroxy-Epoxide 2,3-Epoxy-1,4-dihydroxybutan und 1,2-Epoxy-3,4-dihydroxybutan genannt.

Die Reaktion des Edukts II mit X in der Bedeutung einer CH₂COOY-Gruppe wird in z.B. Wasser, DMF, Dioxan, Alkoholen, Acetonitril, Tetrahydrofuran oder deren Mischungen bei Temperaturen von 0 bis 100 °C, vorzugsweise Raumtemperatur bis 60 °C, bei einem basischen pH-Wert, vorzugsweise bei 9 bis 13, innerhalb von 6 Stunden bis 2 Tagen, vorzugsweise 12 bis 36 Stunden, durchgeführt.

Setzt man einen an den restlichen Stickstoffatomen geschützten Makrocyclus in die Reaktion zur Einführung des R²-Restes ein, so erfolgt die Umsetzung vorzugsweise im Autoklaven in Lösungsmitteln wie zum Beispiel DMF, DMA, Toluol, Methylenchlorid oder deren Gemischen bei Temperaturen von 20 bis 170 °C, vorzugsweise 100 bis 150 °C, unter Zusatz einer Base wie z.B. Aminen, Alkali-, Erdalkalihydroxiden und -carbonate, bevorzugt Kalium- und Natriumcarbonat und -hydroxid, innerhalb von 6 Stunden bis 2 Tagen, vorzugsweise 12 bis 36 Stunden. Verwendet man ein Substrat, das keine nucleofuge Gruppe enthält (das heißt zum Beispiel die oben angeführten Epoxide), so kann auf die Verwendung einer Base verzichtet werden.

Die Verbindungen der allgemeinen Formel I_{A} mit R¹ in der Bedeutung eines Wasserstoffatoms stellen Komplexbildner dar. Sie können isoliert und gereinigt werden oder ohne Isolierung in Metallkomplexe der allgemeinen Formel I_{A} mit mindestens zwei der Substituenten R¹ in der Bedeutung eines Metallionenäquivalents überführt werden.

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat des Elements der Ordnungszahlen 21 - 29, 42, 44, 57 - 83) in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Einführung der gewünschten Metallionen kann dabei sowohl vor als auch nach der Abspaltung der Hydroxyschutzgruppen erfolgen.

Die Neutralisation eventuell noch vorhandener freier Carboxygruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (z.B. durch erschöpfende Umsetzung der freien Carboxy-Gruppen mit Dimethylsulfat).

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder - falls gewünscht - Elektrolyte wie zum Beispiel Natrium-, Calcium-, Magnesium-, Zinkchloride, -phosphate und -citrate oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 0,1 »Mol - 3 Mol/l des Komplexsalzes und werden in der Regel in Mengen von 0,1 »Mol - 5 mMol/kg dosiert. Sie sind zur enteralen und parenteralen Applikation bestimmt. Die erfindungsgemäßen Komplexverbindungen kommen zur Anwendung:
1.) für die NMR- und Röntgen-Diagnostik in Form ihrer Komplexe mit den Ionen der Elemente mit den Ordnungszahlen 21 - 29, 42, 44 und 57-83;
2.) für die Radiodiagnostik und Radiotherapie in Form ihrer Komplexe mit den Radioisotopen der Elemente mit den Ordnungszahlen 27, 29, 31, 32, 37-39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg, vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen können auch in der Radioimmuno- oder Strahlentherapie verwendet werden, Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. [Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der Methode, z.B. Brachytherapie.

Die erfindungsgemäßen therapeutischen Mittel werden parenteral appliziert.

Details der Anwendung von Radiotherapeutika werden z.B. in R.W. Kozak et al. TIBTEC, Oktober 1986, 262, diskutiert.

Die erfindungsgemäßen Mittel sind aufgrund ihrer hervorragenden Wasserlöslichkeit und aufgrund des niedrigen osmotischen Drucks ihrer konzentrierten wäßrigen Lösungen ausgezeichnete Röntgenkontrastmittel, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstands.

### Beispiel 1

### a) 10-(2,3,4-Trihydroxybutyl)-1,4,7-triscarboxymethyl-1,4,7,10-tetraazacyclododecan

10,0 g (28,87 mmol) 1,4,7-Triscarboxymethyl-1,4,7,10-tetraazacyclododecan (DO3A) werden in 40 ml Wasser gelöst und der pH mit 5 normaler Natronlauge auf 13 eingestellt. Man fügt eine Lösung aus 6,24 g (43,30 mmol) 2-(2,2-Dimethyl-1,3-dioxolan-4-yl)-ethylenoxid (DE 3 150 917) in 10 ml Dioxan zu und rührt 24 Stunden bei Raumtemperatur. Es wird mit 60 ml Wasser verdünnt und dreimal mit 50 ml Ether extrahiert. Die wäßrige Phase wird mit 10 %iger Salzsäure auf pH 2 gebracht und eingedampft. Der Rückstand wird in etwas Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und eluiert dann mit 0,5 normaler wäßriger Ameisensaure.

Man dampft im Vakuum ein, löst den Rückstand in wenig heißem Methanol und gibt Aceton hinzu, wobei die Titelverbindung auskristallisiert.

Man erhält 11,31 g (87 % der Theorie) eines weißen Pulvers, das an der Luft zerläuft (laut Analyse 11,1 % Wasser).

| Analyse: (auf Wasser korrigiert) | | | | |
|---|---|---|---|---|
| berechnet: | C 47,99 | H 7,61 | N 12,44 | O 31,97 |
| gefunden: | C 47,93 | H 7,67 | N 12,40 | |

### b) Gadolinium-Komplex von 10-(2,3,4-Trihydroxybutyl)-1,4,7-triscarboxymethyl-1,4,7,10-tetraazacyclododecan

10,0 g (22,2 mmol) der nach 1a) erhaltenen Verbindung werden in 60 ml entionisiertem Wasser gelöst und 4,02 g (11,1 mmol) Gadoliniumoxid zugesetzt. Man erwärmt 3 Stunden auf 90 °C.

Die abgekühlte Lösung wird mit je 2 ml saurem Ionenaustauscher (IR 120) und 2 ml basischem Austauscher (IRA 410) 1 Stunde bei Raumtemperatur gerührt. Man filtriert vom Austauscher ab und kocht das Filtrat mit Aktivkohle kurz auf.

Nach Filtrieren und Gefriertrocknung erhält man 12,76 g (95 % der Theorie) eines weißen amorphen Pulvers (12,3 % Wasser laut Analyse).

| Analyse: (auf Wasser korrigiert) | | | | | |
|---|---|---|---|---|---|
| berechnet: | C 35,73 | H 5,17 | N 9,26 | O 23,8 | Gd 25,99 |
| gefunden: | C 35,68 | H 5,24 | N 9,21 | | Gd 25,93 |

### Beispiel 2

### a) 10-(6-Hydroxy-2,2-dimethyl-1,3-dioxepan-5-yl)-1,4,7-tris(p-toluolsulfonyl)-1,4,7,10-tetraazacyclododecan

50 g (78,76 mmol) 4,7,10-Tris(p-toluolsulfonyl)-1,4,7,10-tetraazacyclododecan und 13,63 g (94,51 mmol) 4,4-Dimethyl-3,5,8-trioxabicyclo-(5.1.0)-octan werden in 300 ml Dimethylformamid gelöst und in einem Autoklaven 24 Stunden auf 170 °C erhitzt. Man dampft zur Trockne ein und chromatographiert den Rückstand an Kieselgel (Laufmittel: Methylenchlorid/Hexan/Aceton: 10/5/1). Die Hauptfraktionen werden eingedampft und aus Methyl-tert.butylether/Methanol umkristallisiert. Ausbeute: 52,76 g (86 % der Theorie) eines cremefarbenen Pulvers.

| Analyse: | | | | |
|---|---|---|---|---|
| berechnet: | C 55,51 | H 6,47 | N 7,19 | S 12,35 |
| gefunden: | C 55,46 | H 6,52 | N 7,18 | S 12,32 |

### b) 10-(6-Hydroxy-2,2-dimethyl-1,3-dioxepan-5-yl)-1,4,7-tetraazacyclododecan

50 g (64,19 mmol) der Titelverbindung aus Beispiel 2a) werden in 800 ml flüssigem Ammoniak / 400 ml Tetrahydrofuran suspendiert und auf -35 °C abgekühlt. Innerhalb von 30 Minuten gibt man 8,9 g (1,28 mol) Lithium zu und rührt 8 Stunden bei -35 °C. Der Überschuß an Lithium wird durch vorsichtige Zugabe von Methanol zerstört. Man läßt das Ammoniakgas vorsichtig abdampfen und dampft anschließend zur Trockne ein. Der Rückstand wird mit 200 ml 4 normaler Natronlauge aufgenommen und dreimal mit 400 ml heißem Toluol extrahiert. Die organischen Phasen werden über Kaliumhydroxid-Plätzchen getrocknet und anschließend im Vakuum eingedampft. Das zurückbleibende Öl wird an Kieselgel chromatographiert (Laufmittel: Methanol/Wasser/konz. Ammoniaklösung = 10/1/1). Man erhält 8,53 g (42 % der Theorie) eines hellgelben Öls, das beim Stehenlassen erstarrt. (8,1 % Wasser laut Analyse).

| Analyse (korrigiert auf Wasser): | | | |
|---|---|---|---|
| berechnet: | C 56,93 | H 10,19 | N 17,71 |
| gefunden: | C 56,88 | H 10,15 | N 17,64 |

### c) 10-(1-Hydroxymethyl-2,3-dihydroxypropyl)-1,4,7-triscarboxymethyl-1,4,7,10-tetraazacyclododecan

8 g (25,28 mmol) der Titelverbindung aus Beispiel 2b) werden in 50 ml Wasser gelöst und 14,05 g (101,12 mmol) Bromessigsäure zugesetzt. Der pH-Wert wird mit 6 normaler Kalilauge auf 9,5 gebracht.

Man erwärmt auf 50 °C und hält den pH-Wert durch Zugabe von 6 n Kalilauge zwischen 9,5 - 10. Nach 12 Stunden Rühren bei 50 °C kühlt man im Eisbad, stellt mit konzentrierter Salzsäure auf pH 2 ein und dampft im Vakuum zur Trockne ein.

Der Rückstand wird in wenig Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und eluiert dann mit 0,5 normaler wäßriger Ameisensäure. Man dampft im Vakuum ein, löst den Rückstand in wenig heißem Methanol und gibt Aceton hinzu. Nach Kühlung im Eisbad kristallisiert die Titelverbindung aus.
- Ausbeute:: 8,56 g (69 % der Theorie) eines hygroskopischen Feststoffs, 9,1 % Wasser laut Analyse).

| Analyse (auf Wasser korrigiert): | | | |
|---|---|---|---|
| berechnet: | C 51,42 | H 7,81 | N 11,42 |
| gefunden: | C 51,37 | H 7,86 | N 11,37 |

### d) Gadolinium-Komplex von 10-(1-Hydroxymethyl-2,3-dihydroxypropyl)-1,4,7-triscarboxymethyl-1,4,7,10-tetraazacyclododecan

8 g (16,31 mmol) der Titelverbindung aus Beispiel 2c) werden in 50 ml entionisiertem Wasser gelöst und 2,96 g (8,15 mmol) Gadoliniumoxid zugesetzt. Man erwärmt 3 Stunden auf 90 °C. Die abgekühlte Lösung wird mit je 2 ml saurem Ionenaustauscher (IR 120) und 2 ml basischem Austauscher (IRA 410) 1 Stunde bei Raumtemperatur gerührt. Man filtriert vom Austauscher ab und kocht das Filtrat kurz mit Aktivkohle auf. Nach Filtration und Gefriertrocknung erhält man 9,99 g (95 % der Theorie) eines amorphen Pulvers (7,8 % Wasser laut Analyse).

| Analyse (korrigiert auf Wasser): | | | | |
|---|---|---|---|---|
| berechnet: | C 39,12 | H 5,47 | N 8,69 | Gd 24,39 |
| gefunden: | C 39,07 | H 5,51 | N 8,61 | Gd 24,32 |

### Beispiel 3

### Dysprosium-Komplex von 10-(2,3,4-Trihydroxybutyl)-1,4,7-triscarboxymethyl-1,4,7,10-tetraazacyclododecan

In analoger Weise zur Vorschrift von 1b) erhält man ausgehend von 1a) mit Dysprosiumoxid anstelle von Gadoliniumoxid den gewünschten Dysprosium-Komplex.

| Analyse: (auf Wasser korrigiert) | | | | |
|---|---|---|---|---|
| berechnet: | C 35,44 | H 5,12 | N 9,19 | Dy 26,64 |
| gefunden: | C 35,38 | H 5,19 | N 9,13 | Dy 26,59 |

### Beispiel 4

### Wismuth-Komplex von 10-(2,3,4-Trihydroxybutyl)-1,4,7-triscarboxymethyl-1,4,7,10-tetraazacyclododecan

In analoger Weise zur Vorschrift von 1b) erhält man ausgehend von 1a) mit Wismuthoxid anstelle von Gadoliniumoxid den entsprechenden Wismuthkomplex.

| Analyse: (auf Wasser korrigiert) | | | | |
|---|---|---|---|---|
| berechnet: | C 32,93 | H 4,76 | N 8,54 | Bi 31,84 |
| gefunden: | C 32,87 | H 4,81 | N 8,49 | Bi 31,78 |

### Beispiel 5

### Ytterbium-Komplex von 10-(1-Hydroxymethyl-2,3-dihydroxypropyl)-1,4,7-triscarboxymethyl-1,4,7,10-tetraazacyclododecan

In analoger Weise zur Vorschrift von 2d) erhält man ausgehend von 2c) mit Ytterbiumoxid anstelle von Gadoliniumoxid den entsprechenden Ytterbium-Komplex.

| Analyse: (auf Wasser korrigiert) | | | | |
|---|---|---|---|---|
| berechnet: | C 34,84 | H 5,04 | N 9,03 | Yb 27,89 |
| gefunden: | C 34,79 | H 5,10 | N 9,01 | Yb 27,83 |

### Beispiel 6

### Lutetium-Komplex von 10-(1-Hydroxymethyl-2,3-dihydroxypropyl)-1,4,7-triscarboxymethyl-1,4,7,10-tetraazacyclododecan

In analoger Weise zur Vorschrift von 2d) erhält man ausgehend von 2c) mit Lutetiumoxid anstelle von Gadoliniumoxid den entsprechenden Lutetium-Komplex.

| Analyse: (auf Wasser korrigiert) | | | | |
|---|---|---|---|---|
| berechnet: | C 34,73 | H 5,02 | N 9,00 | Lu 28,11 |
| gefunden: | C 34,67 | H 4,96 | N 8,96 | Lu 28,06 |

### Beispiel 7

### Europium-¹⁵¹-Komplex von 10-(1-Hydroxymethyl-2,3-dihydroxypropyl)-1,4,7-triscarboxymethyl-1,4,7,10-tetraazacyclododecan

In analoger Weise zur Vorschrift von 2d) erhält man ausgehend von 2c) mit Europiumoxid (¹⁵¹Eu₂O₃) anstelle von Gadoliniumoxid den entsprechenden Europium¹⁵¹-Komplex.

| Analyse: (auf Wasser korrigiert) | | | | |
|---|---|---|---|---|
| berechnet: | C 36,06 | H 5,21 | N 9,35 | Eu 25,35 |
| gefunden: | C 36,01 | H 5,29 | N 9,30 | Eu 25,29 |

### Beispiel 8

### Mangan(II)-Komplex von 10-(1-Hydroxymethyl-2,3-dihydroxypropyl)-1,4,7-triscarboxymethyl-1,4,7,10-tetraazacyclododecan als Natrium-Salz

10 g (22,2 mmol) der Titelverbindung aus Beispiel 2c) werden in 80 ml entionisiertem Wasser gelöst und 2,55 g (22,2 mmol) Mangan(II)carbonat zugesetzt. Es wird 3 Stunden auf 90 °C erhitzt.

Die abgekühlte Lösung wird 1 Stunde mit 10 ml schwach saurem Ionenaustauscher (AMB 252c) bei Raumtemperatur gerührt, Man filtriert vom Austauscher ab.

Das Filtrat wird mit 2N Natronlauge auf pH 7,2 eingestellt und gefriergetrocknet.
- Ausbeute:: 10,75 g (93 % der Theorie) eines farblosen amorphen Pulvers (6,3 % Wasser laut Analyse).

| Analyse: (auf Wasser korrigiert) | | | | | |
|---|---|---|---|---|---|
| berechnet: | C 41,15 | H 5,95 | N 10,66 | Mn 10,46 | Na 4,38 |
| gefunden: | C 41,08 | H 6,03 | N 10,58 | Mn 10,41 | Na 4,43 |

### Beispiel 9

### Herstellung einer Lösung vom Indium-111-Komplex von 10-(1-Hydroxymethyl-2,3-dihydroxypropyl)-1,4,7-triscarboxymethyl-1,4,7,10-tetraazacyclododecan

Eine Lösung von 100 »g des in Beispiel 2c) beschriebenen Komplexbildners in 5 ml eines Gemisches aus 150 mmolaren Kochsalz- und 150 mmolaren Natriumacetat-Lösung (pH 5,8) wird mit 4,5 ml Indium-111-chlorid-Lösung (0,01 mmolar) in 1 ml 0,15 n Salzsäure versetzt und 1 Stunde auf 80 °C erhitzt. Man bringt danach durch Zugabe von 0,1 n Natronlauge die Lösung auf einen pH-Wert von 7,2. Die Lösung wird steril filtriert und lyophilisiert. Der Rückstand wird in physiologischer Kochsalzlösung aufgenommen und stellt dann ein für die Radiodiagnostik geeignetes Präparat dar.

### Beispiel 10

### Herstellung einer Lösung von Gadoliniumkomplex des 1-(1-Hydroxymethyl-2,3-dihydroxypropyl)-4,7,10-triscarboxymethyl-1,4,7,10-tetraazacyclododecans

322,39 g (= 0,5 mol) der in Beispiel 2d) beschriebenen Verbindung werden in 600 ml Wasser pro injectione (p.i.) gelöst. Nach Zugabe von 1,5 g Monohydrat des Calcium-Trinatriumsalzes der DTPA, CaNa₃DTPA und 1,21 g Trishydroxymethylaminomethan stellt man mit verdünnter Salzsäure einen pH-Wert von 7,0 ein und füllt mit Wasser p.i. auf 1000 ml auf. Die Lösung wird ultrafiltriert, in Flaschen abgefüllt und hitzesterilisiert.

### Beispiel 11

### Herstellung einer Lösung vom Yttrium-90-Komplex des 1-(1-Hydroxymethyl-2,3-dihydroxypropyl)-4,7,10-triscarboxymethyl-1,4,7,10-tetraazacyclododecans

Zu einer Lösung von 10 »mol der in Beispiel 2d) beschriebenen Verbindung in 90 »l Ammoniumacetat-Puffer (pH 6,0) werden 5 »l einer Y-90-Lösung (2 »Ci) zugesetzt und das Gemisch 30 Minuten bei 37 °C inkubiert. Man gibt 10 »mol Calcium-Trinatriumsalz der DTPA zu und erhält nach Ultrafiltration ein für die Radiotherapie geeignetes Präparat.

### Beispiel 12

### Herstellung einer Lösung des Gadolinium-Komplexes von 10-(2,3,4-Trihydroxybutyl)-1,4,7-triscarboxymethyl-1,4,7,10-tetraazacyclododecan

967,5 g des in Beispiel 1b) beschriebenen Komplexes werden in 500 ml bidestilliertem Wasser suspendiert. Man bringt durch Zugabe von 1,89 g Natriumbicarbonat auf pH 7,3, fügt 162,3 mg CaNa₂EDTA zu und füllt unter Erwärmen auf ein Volumen von 1 Liter mit bidestilliertem Wasser auf. Nach Filtration durch eine Porenweite von 0,22 »m füllt man die Lösung in Multivials ab und sterilisiert 20 Minuten bei 120 °C. Man erhält ein Kontrastmittel für die Röntgen-Diagnostik.

### Beispiel für eine NMR-diagnostische in-vivo-Untersuchung:

### Darstellung eines Gehirninfarktes bei der Ratte durch den Gadoliniumkomplex von 10-(1-Hydroxymethyl-2,3-dihydroxypropyl)-1,4,7-triscarboxymethyl-1,4,7,10-tetraazacyclododecan (Beispiel 2 d) mit einer Dosis von 0,1 und 0,3 mmol Gd/kg

Bei dem Versuchstier handelte es sich um eine weibliche Wistar-Ratte mit einem Gewicht von 200 g. Für die Induktion des Gehirninfarktes wurde das Tier zunächst betäubt. Nach intravenöser Injektion von Bengalrot mit einer Dosis von 20 mg/kg wurde nahe dem Punkt Bregma ein Bereich des Gehirns von ca. 0,7 cm Durchmesser durch die Schädeldecke hindurch mit Licht der Wellenlänge 548 nm (dies entspricht dem Absorptionsmaximum von Bengalrot) bestrahlt, wodurch es infolge der Bildung von Singulettsauerstoff über eine Kette verschiedener Reaktionen zur Aggregation von Thrombozyten und damit zu einem Infarkt in der bestrahlten Region kommt.

Das Imaging erfolgte in einem MRI-Experimentalgerät der Firma General Electric (Feldstärke 2 Tesla). Es wurde mit einer Spin-Echo-Sequenz (TR = 400 msec, Te = 20 msec) gearbeitet. Die Schichtdicke betrug 3 mm, und es wurden jeweils 4 Mittelungen durchgeführt.

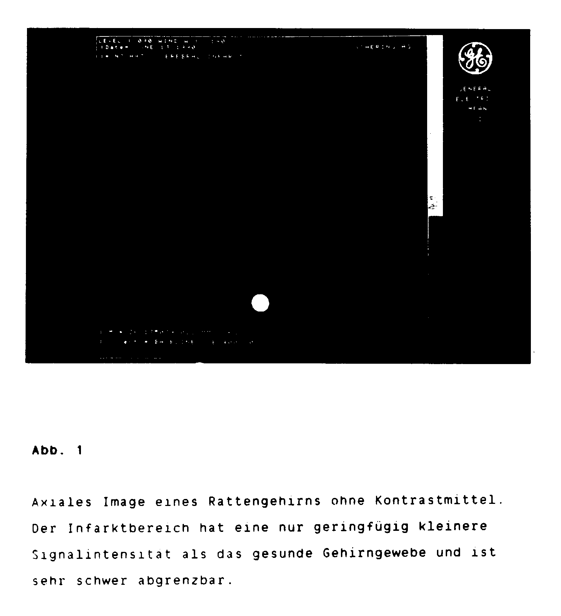

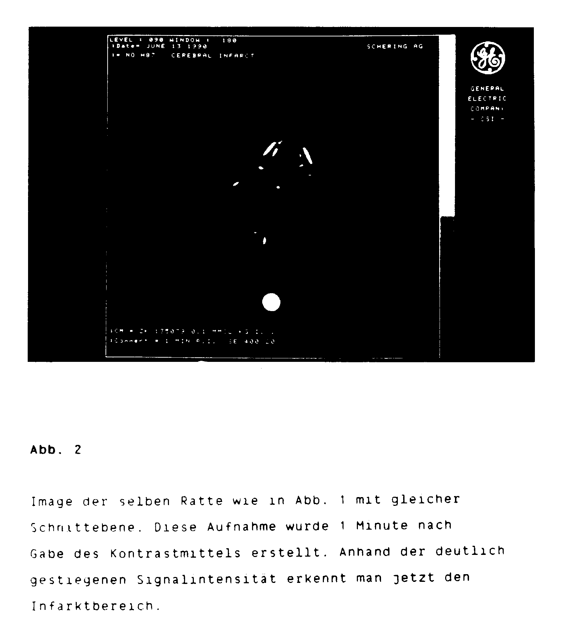

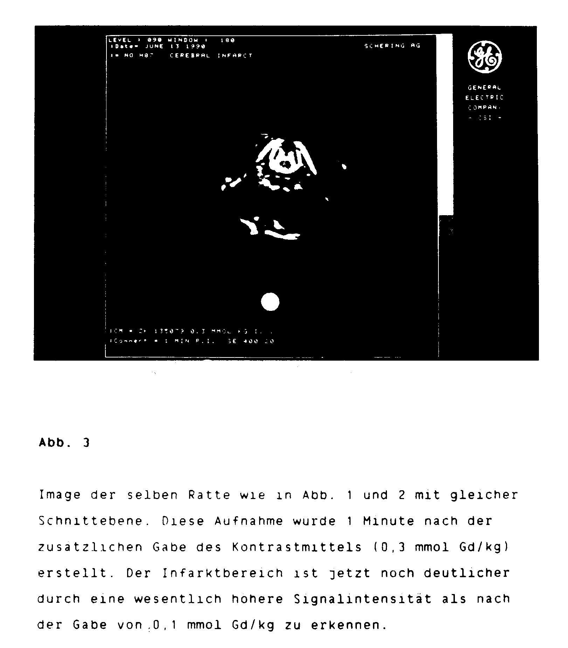

Die Abb. 1 zeigt die Aufnahme ohne Kontrastmittel (axiale Schnittebene). Die Schädeldecke weist nach unten. Der Infarktbereich deutet sich nur ganz schwach durch eine geringere Signalintensität im Vergleich zum gesunden Gewebe an. 1 Minute nach Gabe des Kontrastmittels (0,1 mmol Gd/kg) ergibt sich bereits ein deutliches Enhancement im Bereich des Infarktes und der damit verbundenen Störung der Blut-Hirn-Schranke (Abb. 2). Demselben Tier wurde nach etwa 20 Minuten eine erneute Dosis, diesmal in Höhe von 0,3 mmol Gd/kg, appliziert. 1 Minute nach Applikation zeigt sich deutlich ein wesentlich stärkeres Enhancement als nach der kleineren Dosis, zudem ist der Infarktbereich besser abgrenzbar.

## Patentansprüche

1. 1,4,7,10-Tetraazacyclododecan-butyltriole der allgemeinen Formel I_{A} 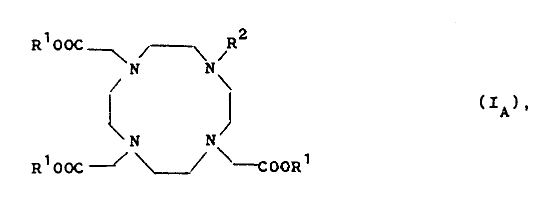 worin
R¹ unabhängig voneinander Wasserstoff oder ein Metallionenäquivalent eines Elements der Ordnungszahlen 21-29, 42, 44 oder 57-83 oder eines Radionuklids eines Elements der Ordnungszahlen 27, 29, 31, 32, 37-39, 43, 49, 62, 64, 70 oder 77 und
R² ein Butyltriol-Rest
bedeuten,
sowie deren Salze mit organischen oder anorganischen Basen oder Aminosäuren.

2. Verbindungen gemäß Anspruch 1, dadurch gekennzeichnet, daß R¹ Wasserstoffatome darstellt.

3. Verbindungen gemäß Anspruch 1, dadurch gekennzeichnet, daß mindestens zwei der Substituenten R¹ Metallionenäquivalente eines Elements der Ordnungszahlen 21-29, 42, 44 oder 57-83 oder eines Radionuklids eines Elements der Ordnungszahlen 27, 29, 31, 32, 37-39, 43, 49, 62, 64, 70 oder 77 sind.

4. 10-(2,3,4-Trihydroxybutyl)-1,4,7-triscarboxymethyl-1,4,7,10-tetraazacyclododecan
10-(1-Hydroxymethyl-2,3-dihydroxypropyl)-1,4,7-triscarboxymethyl-1,4,7,10-tetraazacyclododecan.

5. Gadolinium-Komplexe von
10-(2,3,4-Trihydroxybutyl)-1,4,7-triscarboxymethyl-1,4,7,10-tetraazacyclododecan
10-(1-Hydroxymethyl-2,3-dihydroxpropyl)-1,4,7-triscarboxymethyl-1,4,7,10-tetraazacyclododecan.

6. Pharmazeutische Mittel enthaltend mindestens einen Metallkomplex der allgemeinen Formel I_{A} mit mindestens zwei der Substituenten R¹ in der Bedeutung eines Metallionenäquivalents, gegebenenfalls mit den in der Galenik üblichen Zusätzen.

7. Verwendung von mindestens einem Metallkomplex der allgemeinen Formel I_{A} mit mindestens zwei der Substituenten R¹ in der Bedeutung eines Metallionenäquivalents zur Herstellung von Mitteln für die NMR-, Röntgen-, Ultraschall-, Radio-Diagnostik und Radio-Therapie.

8. Verfahren zur Herstellung von
1,4,7,10-Tetraazacyclododecan-butyltriole der allgemeinen Formel I_{A} 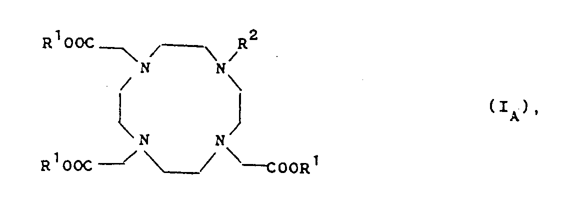 worin
R¹ unabhängig voneinander Wasserstoff oder ein Metallionenäquivalent und
R² ein Butyltriol-Rest
bedeuten,
sowie deren Salze mit organischen oder anorganischen Basen oder Aminosäuren,
dadurch gekennzeichnet, daß man in an sich bekannter Weise Verbindungen der allgemeinen Formel II 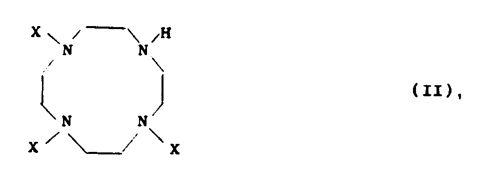 worin
X für eine Stickstoffschutzgruppe oder -CH₂COOY-Gruppe mit Y in der Bedeutung von Wasserstoff, eines Ammoniumkations, eines Alkalimetalls oder einer Schutzgruppe
steht,
mit einem den Rest R² in geschützter Form einführenden Substrat umsetzt, die gegebenenfalls enthaltenen Stickstoffschutzgruppen X entfernt und die so freigesetzten -NH-Gruppen mit einem Essigsäure-Derivat der allgemeinen Formel III
HalCH₂COOY (III)
alkyliert,
worin Hal für Chlor, Brom oder Jod steht,
die (Hydroxy- und gegebenenfalls Säure-) Schutzgruppen entfernt und die so erhaltenen Verbindungen der allgemeinen Formel I_{A} mit R¹ in der Bedeutung von Wasserstoff mit einem Metalloxid oder Metallsalz in die Metallkomplexe der allgemeinen Formel I_{A} mit R¹ in der Bedeutung eines Metallionenäquivalents überführt und anschließend - falls gewünscht - noch vorhandene acide Wasserstoffatome durch Kationen anorganischer und/oder organischer Basen, Aminosäuren oder Aminosäureamide substituiert.

9. Verfahren zur Herstellung der pharmazeutischen Mittel gemäß Anspruch 6, dadurch gekennzeichnet, daß man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

1. 1,4,7,10-tetraazacyclododecanebutyltriols of the general formula I_{A} 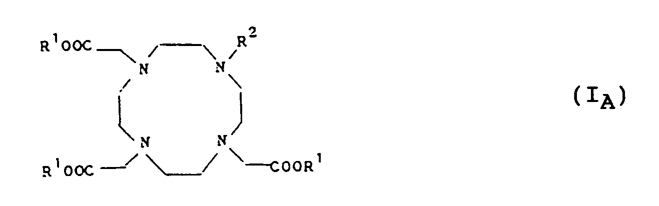 wherein
the substituents R¹, each independently of the others, represent hydrogen or a metal ion equivalent of an element of atomic numbers 21-29, 42, 44 or 57-83 or of a radionuclide of an element of atomic numbers 27, 29, 31, 32, 37-39, 43, 49, 62, 64, 70 or 77, and
R² represents a butyltriol radical,
and their salts with organic or inorganic bases or amino acids.

2. Compounds according to claim 1, characterised in that the substituents R¹ represent hydrogen atoms.

3. Compounds according to claim 1, characterised in that at least two of the substituents R¹ are metal ion equivalents of an element of atomic numbers 21-29, 42, 44 or 57-83 or of a radionuclide of an element of atomic numbers 27, 29, 31, 32, 37-39, 43, 49, 62, 64, 70 or 77.

4. 10-(2,3,4-trihydroxybutyl)-1,4,7-triscarboxymethyl-1,4,7,10-tetraazacyclododecane,
10-(1-hydroxymethyl-2,3-dihydroxypropyl)-1,4,7-triscarboxymethyl-1,4,7,10-tetraazacyclododecane.

5. Gadolinium complexes of
10-(2,3,4-trihydroxybutyl)-1,4,7-triscarboxymethyl-1,4,7,10-tetraazacyclododecane,
10-(1-hydroxymethyl-2,3-dihydroxypropyl)-1,4,7-triscarboxymethyl-1,4,7,10-tetraazacyclododecane.

6. Pharmaceutical agents comprising at least one metal complex of the general formula I_{A}, wherein at least two of the substituents R¹ represent a metal ion equivalent, optionally together with additives customary in galenic pharmacy.

7. Use of at least one metal complex of the general formula I_{A}, wherein at least two of the substituents R¹ represent a metal ion equivalent, for the preparation of agents for NMR, X-ray, ultrasound and radio diagnostics and radiotherapy.

8. Process for the preparation of 1,4,7,10-tetraazacyclododecanebutyltriols of the general formula I_{A} 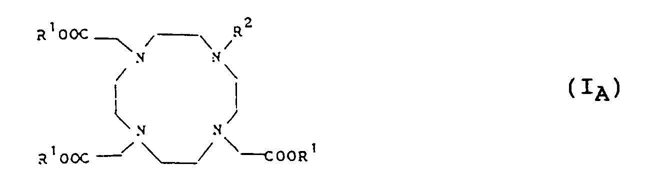 wherein
the substituents R¹, each independently of the others, represent hydrogen or a metal ion equivalent, and
R² represents a butyltriol radical,
and their salts with organic or inorganic bases or amino acids,
characterised in that compounds of the general formula II 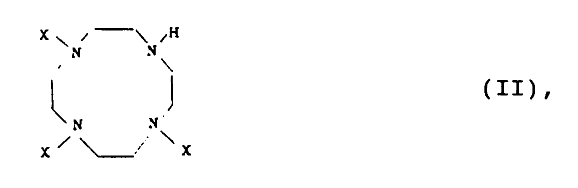 wherein
X represents a nitrogen-protecting group or a group -CH₂COOY, wherein Y represents hydrogen, an ammonium cation, an alkali metal or a protecting group,
are reacted in a manner known per se with a substrate introducing the radical R² in protected form, the nitrogen-protecting groups X which may be present are removed and the -NH- groups so freed are alkylated with an acetic acid derivative of the general formula III
HalCH₂COOY (III),
wherein Hal represents chlorine, bromine or iodine,
the (hydroxy- and optionally acid-) protecting groups are removed and the resulting compounds of the general formula I_{A}, wherein R¹ represents hydrogen, are converted with a metal oxide or metal salt into the metal complexes of the general formula I_{A}, wherein R¹ represents a metal ion equivalent, and then - if desired - any acidic hydrogen atoms still present are substituted by cations of inorganic and/or organic bases, amino acids or amino acid amides.

9. Process for the preparation of the pharmaceutical agents according to claim 6, characterised in that the complex compound dissolved or suspended in water or physiological saline solution, optionally together with additives customary in galenic pharmacy, is brought into a form suitable for enteral or parenteral administration.

## Revendications

1. 1,4,7,10-Tétraazacyclododécane-butyltriols de formule générale I_{A} : 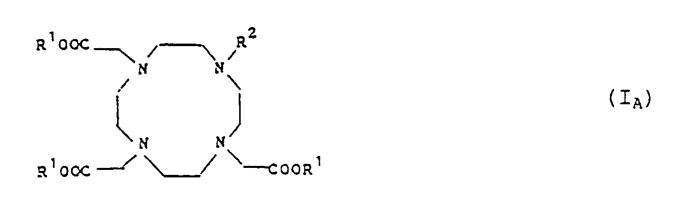 où
les radicaux R¹, indépendamment l'un de l'autre, représentent l'hydrogène ou un équivalent d'ion métallique d'un élément de nombre atomique de 21 à 29, 42, 44 ou de 57 à 83, ou d'un radioisotope d'un élément du nombre atomique 27, 29, 31, 32, 37-39, 43, 49, 62, 64, 70 ou 77, et
R² représente un radical butyltriol,
ainsi que leurs sels avec des bases organiques ou minérales ou des acides aminés.

2. Composés selon la revendication 1, caractérisés en ce que R¹ représente des atomes d'hydrogène.

3. Composés selon la revendication 1, caractérisés en ce qu'au moins deux des substituants R¹ sont des équivalents d'ions de métaux d'un élément avec le nombre atomique de 21 à 29, 42, 44 ou de 57 à 83, ou d'un radioisotope d'un élément ayant le nombre atomique 27, 29, 31, 32, 37-39, 43, 49, 62, 64, 70 ou 77.

4. 10-(2,3,4-Trihydroxybutyl)-1,4,7-triscarboxyméthyl-1,4,7,10-tétraazacyclododécane
10-(1-Hydroxyméthyl-2,3-dihydroxypropyl)-1,4,7-triscarboxyméthyl-1,4,7,10-tétraazacyclododécane.

5. Complexes de gadolinium de 10-(2,3,4-trihydroxybutyl)-1,4,7-triscarboxyméthyl-1,4,7,10-tétraazacyclododécane 10-(1-Hydroxyméthyl-2,3-dihydroxypropyl)-1,4,7-triscarboxyméthyl-1,4,7,10-tétraazacyclododécane.

6. Agent pharmaceutique contenant au moins un complexe métallique de formule générale I_{A} avec au moins deux des substituants R¹ ayant la signification d'un équivalent d'ion métallique, éventuellement avec des adjuvants usuels en galénique.

7. Utilisation d'au moins un complexe métallique de formule générale I_{A} avec au moins deux des substituants R¹ ayant la signification d'un équivalent d'ion métallique pour la préparation d'agents pour le diagnostic par RMN, radiographie à rayons X, ultrasons, radiodiagnostic et radiothérapie.

8. Procédé pour la préparation de 1,4,7,10-tétraazacyclododécane-butyltriol de formule générale I_{A} : 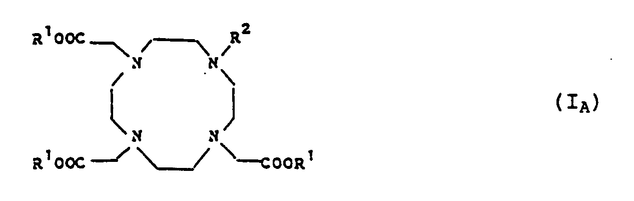 où
les radicaux R¹, indépendamment l'un de l'autre, représentent l'hydrogène ou un équivalent d'ion métallique, et
R² représente un radical butyltriol,
ainsi que leurs sels avec des bases organiques ou minérales ou des acides aminés,
caractérisé en ce qu'on fait réagir de façon connue en soi des composés de formule générale II : 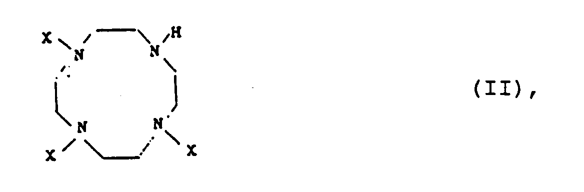 où
X représente un groupe protecteur d'azote ou un groupe -CH₂COOY avec Y ayant la signification d'hydrogène, d'un cation d'ammonium, d'un métal alcalin ou d'un groupe protecteur,
avec un substrat introduisant le radical R² sous forme protégée, en éliminant les groupes protecteurs d'azote X éventuellement contenus et en alkylant les groupes -NH-ainsi libérés avec un dérivé d'acide acétique de formule générale III :
HalCH₂COOY (III)
où Hal représente le chlore, le brome ou l'iode,
en éliminant les groupes protecteurs (d'hydroxy- et éventuellement d'acides) et en transformant les composés ainsi obtenus de formule générale I_{A} avec R¹ ayant la signification de l'hydrogène avec un oxyde métallique ou un sel métallique en complexes de métaux de formule générale I_{A} avec R¹ ayant la signification d'un équivalent d'ion métallique et ensuite - si on le désire - en substituant des atomes d'hydrogène acides encore présents par des cations d'amides d'acides aminés, d'acides aminés ou de bases minérales et/ou organiques.

9. Procédé pour la préparation d'agents pharmaceutiques selon la revendication 6, caractérisé en ce que le composé complexe mis en suspension ou dissous dans l'eau ou dans une solution de sel physiologique, éventuellement avec des adjuvants usuels en galénique, est mis en forme appropriée pour l'application entérale ou parentérale.
